# EUROPÄISCHE PATENTSCHRIFT

(11) **EP 0 659 729 B1**
(45) Veröffentlichungstag und Bekanntmachung des Hinweises auf die Patenterteilung: **24.06.1998**
(21) Anmeldenummer: 94119583.6
(22) Anmeldetag: 10.12.1994
(51) Int. Cl.: C07C 51/58

(54) **Verfahren zur Herstellung von Polyfluorchlor- und Perfluorcarbonsäurechloriden unter Chlorzusatz**
Process for preparing chlorides of polyfluorochloro- and perfluoro carboxylic acids in the presence of chlorine
Procédé de préparation des chlorures d'acides polyfluorochloro- et perfluorocarboxyliques en présence du chlore

(30) Priorität: 23.12.1993 DE 4344241; 15.06.1994 DE 4420763
(43) Veröffentlichungstag der Anmeldung: 28.06.1995
(73) Patentinhaber: Solvay Fluor und Derivate GmbH, D-30173 Hannover (DE)
(72) Erfinder: Braun, Max, Dr., D-30900 Wedemark (DE); Rudolph, Werner, Dr., D-30559 Hannover (DE); Eichholz, Kerstin, D-30855 Langenhagen (DE)
(74) Vertreter: Lauer, Dieter, Dr.

(56) Entgegenhaltungen:
- US-A- 5 259 938
- US-E- R E29 044

## Beschreibung

Die Erfindung bezieht sich auf ein verfahren zur Herstellung von Polyfluorchlor- und Perfluorcarbonsäurechloriden, insbesondere auf ein verfahren zur Herstellung von Trifluoracetylchlorid, Chlordifluoracetylchlorid und Perfluorpropionylchlorid, unter Zusatz von elementarem Chlor.

Polyfluorchlor- und Perfluorcarbonsäurechloride sind Zwischenprodukte in der chemischen Synthese, z. B. bei der Herstellung von Pharmazeutika und Agrochemikalien.

Chlordifluoracetylchlorid ist ein Zwischenprodukt, welches in mannigfaltiger Weise in der chemischen Synthese eingesetzt werden kann, beispielsweise in der Farbstoffherstellung. Alkyl- und Arylhalogenide können durch ein Derivat von Chlordifluoracetylchlorid, nämlich durch den Methylester, in Anwesenheit von Kaliumfluorid und Kupferjodid trifluormethyliert werden. Bislang wurde Chlordifluoracetylchlorid durch Solvolyse von CF₂ClCCl₃ mit Oleum oder SO₃ in Anwesenheit von Quecksilberverbindungen und Chlorschwefeloxiden hergestellt, wie in der DE-OS 19 17 630 beschrieben. Der erwähnte Methylester ist auch Vorprodukt für die Herstellung von Difluorcarben, siehe G. A. Wheaton und D. J. Donald in J. Fluorine Chem. 8 (1976), Seiten 97 bis 100. Difluorcarben wird bei der Insektizid-Herstellung eingesetzt, siehe EP-A 198 791 (US-A 4,701,563). Die Herstellung von Difluorcarben aus Trifluormethyl-Phenylquecksilber und anderen Verbindungen dieser Art ist aus Umweltgesichtspunkten problematisch.

US-A-5259938 beschreibt ein Verfahren zur Herstellung von *inter alia* Trifluoracetylchlorid und Pentafluorpropionylchlorid, wobei CF₂CHCl₂ bzw. CF₃CF₂CHCl₂ mit Sauerstoff unter Zusatz von elementarem Chlor in der flüssigen Phase mit Licht einer Wellenlänge >280nm bestrahlt wird.

Trifluoracetylchlorid ist ebenfalls ein wichtiges Zwischenprodukt in der chemischen Synthese. Die Umsetzung mit Trifluorethanol führt zum entsprechenden Ester, der durch Hydrierung in zwei Moleküle Trifluorethanol gespalten werden kann. Trifluorethanol ist ein Lösungsmittel, das auch in Trocknungs- und Reinigungsverfahren eingesetzt werden kann.

Perfluorpropionylchlorid ist Zwischenprodukt in der chemischen Synthese. So kann es zu 2,2,3,3,3-Pentafluorpropanol hydriert werden; diese Verbindung eignet sich im Gemisch mit 1-Chlor-2,2,2-trifluorethyl-difluorethylether als Kühlschmiermittel oder in Reinigungs- und Trocknungsverfahren, siehe DE-OS 42 27 130.

Aufgabe der vorliegenden Erfindung ist es, ein Verfahren zur Herstellung von Polyfluorchlorcarbonsäurechloriden und Perfluorcarbonsäurechloriden, insbesondere zur Herstellung von Trifluoracetylchlorid, Chlordifluoracetylchlorid und Perfluorpropionylchlorid, anzugeben, welches ohne Zusatz von Quecksilberverbindungen durchgeführt werden kann und hohe Ausbeuten bei hoher Selektivität und hohen Reaktionsgeschwindigkeiten ergibt. Diese Aufgabe wird durch das Verfahren der vorliegenden Erfindung gelöst.

Das erfindungsgemäße Verfahren zur Herstellung von Verbindungen der Formel RCFXC(O)Cl, worin R für Fluor oder perfluoriertes Alkyl mit 1 bis 10 C-Atomen und X für Chlor oder Fluor steht, sieht vor, daß man Verbindungen der Formel RCFXCHCl₂, worin R und X die obengenannte Bedeutung aufweisen, kontinuierlich mit Sauerstoff in der Gasphase unter Zusatz von elementarem Chlor umsetzt, wobei man eine aktivierende Bestrahlung nur mit Licht einer Wellenlänge λ ≥ 290 nm vornimmt. R bedeutet vorzugsweise Fluor, CClF₂ oder perfluoriertes C1 - C3-alkyl, insbesondere Fluor, CClF₂, perfluoriertes Methyl oder perfluoriertes Ethyl.

Zur Bestrahlung kann man beispielsweise Bestrahlungslampen (z. B. Philips-Leuchtstoff-Röhren) verwenden, die nur (UV-)Licht einer Wellenlänge oberhalb von 290 nm (λ ≥ 290 nm) abstrahlen. Hier ist die Bestrahlung durch Quarzglas möglich. Einzige Voraussetzung für diese Variante ist, daß diese Lampen im Absorptionsbereich des elementaren Chlor emittieren. Alternativ dazu kann man auch beispielsweise Bestrahlungslampen (z. B. Hg-Mittel- oder Hochdruckstrahler) verwenden, die auch einige Linien im Bereich unterhalb von 290 nm (λ < 290 nm) emittieren. Bei dieser Variante muß durch ein Glas bestrahlt werden, das nur für Licht einer Wellenlänge von 290 nm oder darüber (λ ≥ 290 nm) durchlässig ist, also den kürzerwelligen Bestrahlungsanteil mit λ < 290 nm herausfiltert. Gut geeignet dafür sind beispielsweise Borosilicat-Gläser. Derartige Gläser enthalten üblicherweise 7 bis 13 % B₂O₃, 70 bis 80 % SiO₂, ferner 2 bis 7 % Al₂O₃ und 4 bis 8 % Na₂O + K₂O sowie 0 bis 5 % Erdalkalimetalloxide. Bekannte Warenzeichen für Borosilicat-Gläser sind Duran, Pyrex und Solidex. Selbstverständlich kann man auch so vorgehen, daß man einerseits eine Bestrahlungslampe einsetzt, die Licht oberhalb der angegebenen Wellenlänge abstrahlt, und zusätzlich Gläser verwendet, die für Licht oberhalb der angegebenen Wellenlänge durchlässig sind (d. h. für Licht unterhalb der angegebenen Wellenlänge entsprechend undurchlässig sind).

Gut geeignet zur Bestrahlung sind auch Hg-Hochdruck-Lampen, die aufgrund eines Dotierungsmittel überwiegend oder nur im Wellenbereich bei oder oberhalb von 290 nm abstrahlen. Hg-Hochdruckstrahler beispielsweise weisen eine recht intensive Bande im Bereich von 254 nm auf, die, wie oben beschrieben worden ist, z. B. durch Borosilikat-Glas herausgefiltert wird. Bei durch Metalliodide dotierten Hg-Hochdruckstrahlern ist diese Linie stark unterdrückt. Überraschend ist die oft überproportionale Erhöhung der Umsatzrate bei solchen dotierten Strahlern. Hervorragende Ergebnisse im Hinblick auf Umsatzrate und Selektivität erzielt man mit Hg-Hochdruckstrahlern, die mit Galliumiodid dotiert sind, und besonders mit Strahlern, die mit Thalliumiodid oder Cadmiumiodid dotiert sind. Auch bei Verwendung solcher Strahler setzt man vorteilhaft Glas ein, das den kürzerwelligen Strahlungsanteil mit λ < 290 nm herausfiltert.

Zweckmäßig arbeitet man in bezug auf Umsetzungstemperatur und Druck derart, daß es zu keiner Kondensation auf den Apparatebauteilen kommt. Vorzugsweise führt man die Umsetzung bei bis zu 200 °C, vorzugsweise in einem Temperaturbereich von 50 bis 130 °C durch. Man kann bei vermindertem Druck arbeiten, vorzugsweise arbeitet man bei einem Druck von 1 bis 10 atm (abs.). Ganz besonders bevorzugt arbeitet man drucklos. Der Begriff "drucklos" bedeutet im Rahmen der vorliegenden Erfindung, daß auf die Reaktionsmischung außer dem Umgebungsdruck (d. h. etwa 1 atm), dem Förderdruck des Sauerstoffgases (bzw. des sauerstoffhaltigen Gases, man kann z. B. Luft einsetzen) und des Chlors und dem sich gegebenenfalls ausbildenden Druck durch bei der Reaktion entstehendes Chlorwasserstoffgas kein zusätzlicher Druck einwirkt.

Das Verfahren wird kontinuierlich durchgeführt, wobei man die Umsetzung vorteilhaft in einer Durchflußapparatur durchführt. Vorzugsweise geht man so vor, daß man kontinuierlich Ausgangsmaterial (die entsprechende Wasserstoff und Halogen enthaltende Ausgangsverbindung, Chlor und Sauerstoff) in die Durchflußapparatur einspeist und entsprechend der eingespeisten Menge kontinuierlich Reaktionsprodukt abzieht.

Das Molverhältnis zwischen der Ausgangsverbindung RCFXCHCl₂ und dem elementaren Chlor kann in einem weiten Bereich schwanken z. B. von 100:1 bis 1:1. Besonders gute Ergebnisse werden erzielt, wenn das Molverhältnis zwischen der Ausgangsverbindung und dem elementaren Chlor im Bereich von 100:1 bis 3:1, vorzugsweise 50:1 bis 5:1 liegt.

Auch das Molverhältnis zwischen der Ausgangsverbindung RCFXCHCl₂ und Sauerstoff kann in einem weiten Bereich schwanken. Zweckmäßig setzt man mindestens 0,5 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,5 bis 1:20, insbesondere 1:1,1 bis 1:3 liegt. Der Sauerstoff kann in Form von Luft oder als 0₂/Inertgas-Gemisch, vorzugsweise aber als reiner Sauerstoff eingesetzt werden.

Die benötigten Ausgangsmaterialien der Formel RCFXCHCl₂ sind bekannt oder nach Standardmethoden herstellbar. Die Herstellung von 1.1.1.2.2.-Pentafluoro-3.3-dichloropropan (HCFC 225ca) aus 1.1.1.2.2.-Pentafluoro-3.3.3-trichloropropan und Wasserstoff über Iridiumkatalysatoren wird beispielsweise in der japanischen Offenlegungsschrift 04/210 653 beschrieben. Das genannte HCFC 225ca wird dann zu Perfluorpropionsäurechlorid nach dem erfindungsgemäßen Verfahren weiterverarbeitet.

Eine besonders bevorzugte Ausführungsform betrifft die Herstellung von Trifluoracetylchlorid, Chlordifluoracetylchlorid und Perfluorpropionylchlorid.

Diese bevorzugte Ausführungsform umfaßt die Herstellung von Verbindungen der allgemeinen Formel CF₂XC(O)Cl, worin X = CF₃, Cl oder F bedeutet, und ist dadurch gekennzeichnet, daß man HCFC 225ca, 1.1-Difluor-1.2.2-trichlorethan (HCFC 122) bzw. 1.1.1-Trifluor-2.2-dichlorethan (HCFC 123) mit Sauerstoff in der Gasphase unter Zusatz von elementarem Chlor unter Bestrahlung mit Licht einer Wellenlänge λ ≥ 290 nm umsetzt. Anhand dieser Ausführungsform wird die Erfindung weiter erläutert.

Die aktivierende Bestrahlung wird vorzugsweise mit Strahlern durchgeführt, die Licht abgeben, das mindestens teilweise im UV-Bereich liegt. Beispielsweise sind Hg-Hochdruck-und Mitteldruckstrahler geeignet. Auch Leuchtstoffröhren, z. B. Philips-Leuchtstoffröhren mit selektiver Emission bei 350 nm, sind einsetzbar. Als Werkstoff für die entsprechenden Apparaturbestandteile empfiehlt sich UV-durchlässiges Material. Quarz kann, wie gesagt, verwendet werden, sofern die Strahler Licht bei oder oberhalb einer Wellenlänge von 290 nm abstrahlen. Anderenfalls oder alternativ setzt man die genannten Borosilicat-Gläser ein.

In bezug auf die Produktreinheit ist es wünschenswert, daß möglichst wenig Wasser bei der Umsetzung vorhanden ist. Gewünschtenfalls kann man die Reaktanten in bekannter Weise von mitgeschlepptem Wasser befreien, beispielsweise durch Kontaktieren mit Trocknungsmitteln wie Trockenperlen, Phosphorpentoxid oder hochkonzentrierter Schwefelsäure.

Die durchschnittliche Verweilzeit im Reaktionsgefäß liegt vorzugsweise zwischen 0,1 und 30 Minuten. Die optimale durchschnittliche Verweilzeit, die u. a. von der Lampenleistung und von geometrischen Parametern der Bestrahlungsapparatur (Durchflußapparatur) abhängig ist, kann man durch einfache Handversuche und Analyse des Produktstromes, z. B. durch Gaschromatographie, ermitteln.

Bessere Umsatzraten und höhere Selektivität können erzielt werden, wenn statt einer einzelnen Bestrahlungslampe mit bestimmter Leistung zwei oder mehr leistungsschwächere Lampen gleicher Gesamtleistung in hintereinandergeschalteten Reaktoren eingesetzt werden. Auch ist eine gute Verwirbelung der Reaktionsmischung, z. B. durch geeignete Einbauten im Reaktor, oft von Vorteil.

Das Verfahren bietet einige überraschende Vorteile. Beispielsweise wird, sofern man Borosilicat-Glas verwendet, dieses nicht oder allenfalls in extrem geringem Umfang von aggressiven Reaktionsprodukten angegriffen. Überraschend ist auch die hohe Umsatzrate bei hoher Selektivität trotz des obligatorischen Einsatzes von elementarem Chlor (man findet keine bzw. lediglich Spuren chlorierter Nebenprodukte), insbesondere bei Anwendung von mit Metalliodid dotierten Hg-Hochdruckstrahlern.

Aus der Veröffentlichung von E. O. Edney, B. W. Gay Jr. und D. J. Driscoll in J. Atmos. Chem. 12 (2) (1991), Seiten 105 bis 120 ist zwar eine wissenschaftliche Untersuchung bereits bekannt, in welcher durch eine Fourier-Transform-IR-Methode die Oxidation von HCFC 123 in Anwesenheit von Chlor untersucht wurde. Dabei wurde die Untersuchung bei Unterdruck in einer zufällig aus Pyrex-Glas bestehenden IR-Meßzelle durchgeführt. Erst die Untersuchung durch die Erfinder der vorliegenden Anmeldung führte zu der Erkenntnis, daß die Bestrahlung mit Licht einer Wellenlänge λ ≥ 290 nm gezielt die technisch vorteilhafte, insbesondere bei Einsatz dotierter Strahler mit hoher Selektivität und Ausbeute ablaufende Anwendung eines solchen Verfahrens zur Herstellung von bestimmten Carbonsäurechloriden ermöglicht.

Die folgenden Beispiele sollen die Erfindung weiter erläutern, ohne sie in ihrem Umfang einzuschränken (Beispiel 1 ist ein Vergleichsbeispiel, die übrigen Beispiele sind erfindungsgemäß).

### Beispiel 1: (Vergleichsbeispiel)

Kontinuierliche Herstellung von Chlordifluoracetylchlorid durch photochemische Oxidation von CF₂Cl-CHCl₂ (HCFC 122) mit Sauerstoff durch Quarzglas und Chlor als Sensibilisator 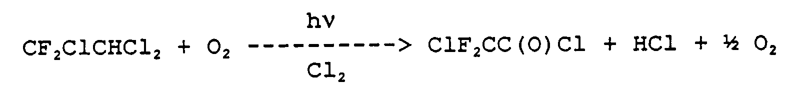 In einem 400 ml Photolyse-Tauchschachtreaktor wurde bei 100 °C Reaktorinnentemperatur eine Mischung aus CF₂Cl-CHCl₂ (aus Vorverdampfer mit T = 150 °C) und reinem Sauerstoff im Molverhältnis 1:1,4 gasförmig zusammen mit 10 Mol-% Cl₂ (bezogen auf HCFC 122) zudosiert und währenddessen mit einem Hg-Hochdruckstrahler TQ 718 von Heraeus (Einstellung 500 W) durch Quarzglas bestrahlt. Die Dosierung an HCFC 122 betrug 0,91 mol/30 min. Der den Reaktor verlassende Gasstrom enthielt an Produkten 72,4 % Chlordifluoracetylchlorid und 4,7 % 1,1-Difluortetrachlorethan (112a). Der Umsatz an 122 betrug 99 %, die Selektivität 72 %.

### Beispiel 2:

Kontinuierliche Herstellung von Chlordifluoracetylchlorid durch photochemische Oxidation von CF₂Cl-CHCl₂ (HCFC 122) mit Sauerstoff durch Borosilicat-Glas und Chlor als Sensibilisator 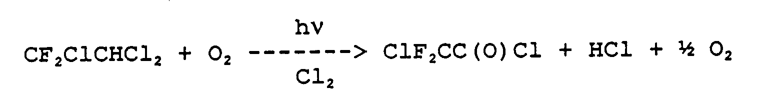 In einem 400 ml Photolyse-Tauchschachtreaktor wurde bei 100 °C Reaktorinnentemperatur eine Mischung aus CF₂Cl-CHCl₂ (HCFC 122; aus Vorverdampfer mit T = 150 °C) und reinem Sauerstoff im Molverhältnis 1:1,4 gasförmig zusammen mit 10 Mol-% Cl₂ (bezogen auf HCFC 122) zudosiert und währenddessen mit einem Hg-Hochdruckstrahler TQ 718 von Heraeus (Einstellung 700 W) durch Pyrex^{R}-Glas bestrahlt. Die Dosierung an HCFC 122 betrug 0,91 mol/30 min. Der den Reaktor verlassende Gasstrom enthielt an Produkten 93,0 % Chlordifluoracetylchlorid neben 0,3 % 1,1-Difluortetrachlorethan (112a). Der Umsatz an HCFC 122 betrug 69 %, die Selektivität 93 %.

### Beispiel 3:

Ansatz und Durchführung wie Beispiel 2, jedoch mit 24 Mol-% Chlorgas (bezogen auf HCFC 122). Der den Reaktor verlassende Gasstrom enthielt an Produkten 92,8 % Chlordifluoracetylchlorid neben 1,0 % 1,1-Difluortetrachlorethan (HCFC 112a). Der Umsatz an HCFC 122 betrug 94 %, die Selektivität 93 %.

### Beispiel 4:

Beispiel 2 wurde unter Verwendung einer Apparatur aus Duran-50^{R}-Glas wiederholt. Die Ergebnisse entsprachen denen von Beispiel 2.

### Beispiel 5:

Beispiel 3 wurde unter Verwendung einer Apparatur aus Duran-50^{R}-Glas wiederholt. Die Ergebnisse entsprachen denen von Beispiel 3.

### Beispiel 6:

Kontinuierliche Herstellung von Trifluoracetylchlorid durch photochemische Oxidation von CF₃-CHCl₂ (HCFC 123) mit Sauerstoff durch Borosilicat-Glas und Chlor als Sensibilisator 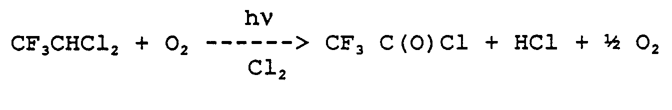 In einem 400 ml Photolyse-Tauchschachtreaktor wurde bei 100 °C Reaktorinnentemperatur eine Mischung aus CF₃-CHCl₂ (aus Vorverdampfer mit T = 100 °C) und reinem Sauerstoff im Molverhältnis 1:1,2 gasförmig zusammen mit 38 Mol-% Cl₂ (bezogen auf HCFC 123) zudosiert und währenddessen mit einem Hg-Hochdruckstrahler TQ 718 von Heraeus (Einstellung 700 W) durch Pyrex^{R}-Glas bestrahlt. Die Dosierung an HCFC 123 betrug 0,96 mol/30 min. Der den Reaktor verlassende Gasstrom enthielt an Produkten 98,6 % Trifluoracetylchlorid neben 1,1 % 1,1,1-Trichlortrifluorethan (113a). Der Umsatz an HCFC 123 betrug 71 %, die Selektivität 99 %.

### Beispiel 7:

Ansatz und Durchführung wie Beispiel 6, jedoch mit 16 Mol-% Chlorgas (bezogen auf HCFC 123) und einem molaren Verhältnis HCFC 123/0₂ = 1:1,7. Der Umsatz lag bei 96 %, die Selektivität betrug 97 %.

### Beispiel 8:

Beispiel 6 wurde mit einer Apparatur aus Duran-50^{R}-Glas wiederholt. Die Ergebnisse entsprachen denen von Beispiel 6.

### Beispiel 9:

Beispiel 7 wurde mit einer Apparatur aus Duran-50^{R}-Glas wiederholt. Die Ergebnisse entsprachen denen von Beispiel 7.

### Beispiel 10:

Herstellung von Trifluoracetylchlorid durch photochemische oxidation von CF₃CHCl₂ mit Sauerstoff durch Duran (Pyrex)-Glas unter Verwendung von dotierten Strahlern als Lichtquellen

### Beispiel 10.1:

Strahlungsquelle: Mit Galliumiodid dotierter Hg-Hochdruckstrahler 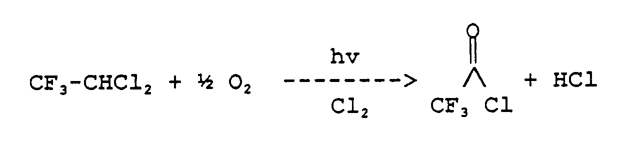 In einen 400 ml Photolyse-Tauchschachtreaktor wurde bei 100 °C Reaktorinnentemperatur eine Mischung aus CF₃-CHCl₂ (aus Vorverdampfer mit T = 100 °C) und reinem Sauerstoff im Molverhältnis 1:1,17 gasförmig zusammen mit 20 Mol-% Cl₂ (bezogen auf 123) zudosiert und währenddessen mit einem Hg-Hochdruckstrahler **TQ 718 Z 1** von Heraeus (Einstellung 500 W) durch Pyrex- bzw. Duran 50-Glas bestrahlt. Die Dosierung an 123 betrug 0,96 mol/30 min. Die Selektivität der Reaktion lag bei 100 % (GC-Analyse), der Umsatz betrug (mit Einstellung der Lampenleistung auf 500 W) 45 %.

### Beispiel 10.2:

Strahlungsquelle: Mit Thalliumiodid dotierter Hg-Hochdruckstrahler 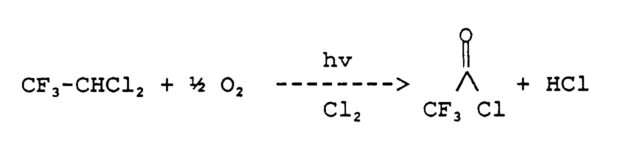 In einen 400 ml Photolyse-Tauchschachtreaktor wurde bei 100 °C Reaktorinnentemperatur eine Mischung aus CF₃-CHCl₂ (aus Vorverdampfer mit T = 100 °C) und reinem Sauerstoff im Molverhältnis 1:1,18 gasförmig zusammen mit 20 Mol-% Cl₂ (bezogen auf 123) zudosiert und währenddessen mit einem Hg-Hochdruckstrahler **TQ 718 Z 2** von Heraeus (Einstellung 500 W) durch Pyrex- bzw. Duran 50-Glas bestrahlt. Die Dosierung an 123 betrug 0,96 mol/30 min. Die Selektivität der Reaktion lag bei 98 %, der Umsatz betrug (mit Einstellung der Lampenleistung auf 500 W) 83 %.

### Beispiel 10.3:

Strahlungsquelle: Mit Cadmiumiodid dotierter Hg-Hochdruckstrahler 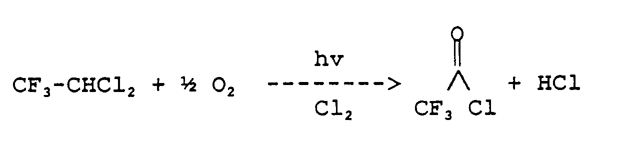 In einen 400 ml Photolyse-Tauchschachtreaktor wurde bei 100 °C Reaktorinnentemperatur eine Mischung aus CF₃-CHCl₂ (aus Vorverdampfer mit T = 100 °C) und reinem Sauerstoff im Molverhältnis 1:1,23 gasförmig zusammen mit 20 Mol-% Cl₂ (bezogen auf 123) zudosiert und währenddessen mit einem Hg-Hochdruckstrahler **TQ 718 Z 3** von Heraeus (Einstellung 500 W) durch Pyrex- bzw. Duran 50-Glas bestrahlt. Die Dosierung an 123 betrug 0,96 mol/30 min. Die Selektivität der Reaktion lag bei 100 %, der Umsatz betrug (mit Einstellung der Lampenleistung auf 500 W) ebenfalls 100 %.

Den Beispielen 10.1 bis 10.3 kann entnommen werden, daß trotz verringerter Lampenleistung bei Verwendung dotierter Strahler eine erhebliche Umsatzsteigerung zu beobachten ist.

### Beispiel 11:

Herstellung von Trifluoracetylchlorid mit nicht dotiertem Strahler bei verringerter Lampenleistung
Beispiel 10.1 wurde analog wiederholt. Diesmal wurde jedoch der nicht dotierte Hg-Hochdruckstrahler TQ 718 von Heraeus eingesetzt; die Lampenleistung betrug 500 W wie in Beispiel 10.1. Die Selektivität lag bei 100 % (GC), der Umsatz lag bei 41 %.

### Beispiel 12:

Herstellung von Pentafluorpropionsäurechlorid durch photochemische Oxidation von CF₃CF₂CHCl₂ (225ca) mit Sauerstoff durch Duran-Glas und Chlor als Sensibilisator 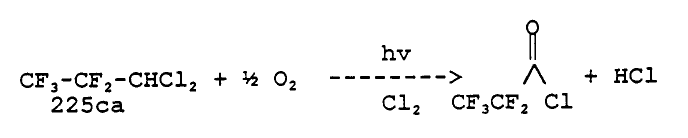 In einen 400 ml Photolyse-Tauchschachtreaktor wurde bei 100 °C Reaktorinnentemperatur eine Mischung aus CF₃CF₂CHCl₂ (98 % Reinheit; aus Vorverdampfer mit T = 130 °C) und reinem Sauerstoff im Molverhältnis 1:1,6 gasförmig zusammen mit 20 Mol-% Cl₂ (bezogen auf 225ca) zudosiert und währenddessen mit einem Hg-Hochdruckstrahler TQ 718 von Heraeus (Einstellung 700 W) durch Pyrex- bzw. Duran 50-Glas bestrahlt. Die Dosierung an 225ca betrug 0,25 mol/15 min. Der den Reaktor verlassende Gasstrom enthielt gemäß Gaschromatogramm 96,2 % Pentafluorpropionsäurechlorid, bezogen auf den als 100 % gesetzten Gesamtgehalt an kohlenstoffhaltigen Produkten. Die Selektivität betrug somit 96,2 %, der Umsatz lag bei 61,5 %.

Die Aufarbeitung der Reaktionsprodukte (Abtrennung von Chlor) erfolgte bei allen Versuchen entweder durch Einleiten des Reaktor-Gasstromes in Alkohol (Veresterung der Säurechloride) oder durch fraktionierende Feindestillation (Trifluoracetylchlorid zweckmäßig in einer Druckkolonne).

## Patentansprüche

1. Verfahren zur Herstellung von Verbindungen der Formel RCFXC(O)Cl, worin R für Fluor oder perfluoriertes Alkyl mit 1 bis 10 C-Atomen und X für Chlor oder Fluor steht, wobei man Verbindungen der Formel RCFXCHCl₂, worin R und X die obengenannte Bedeutung aufweisen, kontinuierlich mit Sauerstoff in der Gasphase unter Zusatz von elementarem Chlor umsetzt, wobei man eine aktivierende Bestrahlung nur mit Licht einer Wellenlänge λ ≥ 290 nm vornimmt.

2. Verfahren nach Anspruch 1, dadurch gekennzeichnet, daß man die Umsetzung in einer Durchflußapparatur durchführt.

3. Verfahren nach Anspruch 2, dadurch gekennzeichnet, daß man kontinuierlich Ausgangsmaterial einspeist und Reaktionsprodukt abzieht.

4. Verfahren nach einem der vorhergehenden Ansprüche, dadurch gekennzeichnet, daß man drucklos arbeitet.

5. Verfahren nach einem der vorhergehenden Ansprüche, dadurch gekennzeichnet, daß das Molverhältnis zwischen der Ausgangsverbindung RCFXCHCl₂ und elementarem Chlor im Bereich von 100:1 bis 3:1 liegt.

6. Verfahren nach einem der vorhergehenden Ansprüche, dadurch gekennzeichnet, daß das Molverhältnis zwischen der Ausgangsverbindung RCFXCHCl₂ und Sauerstoff (O₂) im Bereich von 1:0,5 bis 1:20 liegt.

7. Verfahren nach einem der vorhergehenden Ansprüche, dadurch gekennzeichnet, daß man zur Herstellung von Trifluoracetylchlorid ausgeht von 1.1.1-Trifluor-2.2-dichlorethan (HCFC123), zur Herstellung von Chlordifluoracetylchlorid von 1.1-Difluor-1.2.2-trichlorethan (HCFC122) oder zur Herstellung von Pentafluorpropionylchlorid von 1.1.1.2.2-Pentafluor-3.3-dichlorpropan (HCFC225ca) ausgeht.

8. Verfahren nach einem der vorhergehenden Ansprüche, dadurch gekennzeichnet, daß man die Umsetzung bei einer Temperatur von bis zu 200 °C, vorzugsweise in einem Temperaturbereich von 50 bis 130 °C durchführt.

9. Verfahren nach einem der Ansprüche 2 bis 8, dadurch gekennzeichnet, daß die durchschnittliche Verweilzeit in der Durchflußapparatur zwischen 0,1 und 30 Minuten liegt.

10. Verfahren nach einem der vorhergehenden Ansprüche, dadurch gekennzeichnet, daß man einen mit einem Metalliodid dotierten Hg-Hochdruckstrahler einsetzt.

11. Verfahren nach Anspruch 10, dadurch gekennzeichnet, daß man einen mit Galliumiodid, Thalliumiodid oder Cadmiumiodid dotierten Hg-Hochdruckstrahler einsetzt.

## Claims

1. Process for the preparation of compounds of the formula RCFXC(O)Cl, in which R is fluorine or perfluorinated alkyl having 1 to 10 C atoms and X is chlorine or fluorine, wherein compounds of the formula RCFXCHCl₂, in which R and X have the abovementioned meaning, are reacted continuously with oxygen in the gas phase with addition of elemental chlorine, an activating irradiation only with light of a wavelength λ ≥ 290 nm being carried out.

2. Process according to Claim 1, characterized in that the reaction is carried out in a flow apparatus.

3. Process according to Claim 2, characterized in that starting material is continuously fed in and reaction product is drawn off.

4. Process according to one of the preceding claims, characterized in that the procedure is pressureless.

5. Process according to one of the preceding claims, characterized in that the molar ratio between the starting compound RCFXCHCl₂ and elemental chlorine is in the range from 100:1 to 3:1.

6. Process according to one of the preceding claims, characterized in that the molar ratio between the starting compound RCFXCHCl₂ and oxygen (O₂) is in the range from 1:0.5 to 1:20.

7. Process according to one of the preceding claims, characterized in that 1,1,1-trifluoro-2,2-dichloroethane (HCFC 123) is used as a starting material for the preparation of trifluoroacetyl chloride, 1,1-difluoro-1,2,2-trichloroethane (HCFC 122) for the preparation of chlorodifluoroacetyl chloride or 1,1,1,2,2-pentafluoro-3,3-dichloropropane (HCFC 225ca) for the preparation of pentafluoropropionyl chloride.

8. Process according to one of the preceding claims, characterized in that the reaction is carried out at a temperature of up to 200°C, preferably in a temperature range from 50 to 130°C.

9. Process according to one of Claims 2 to 8, characterized in that the average residence time in the flow apparatus is between 0.1 and 30 minutes.

10. Process according to one of the preceding claims, characterized in that an Hg high-pressure radiator doped with a metal iodide is employed.

11. Process according to Claim 10, characterized in that an Hg high-pressure radiator doped with gallium iodide, thallium iodide or cadmium iodide is employed.

## Revendications

1. Procédé de préparation de composés de formule RCFXC(O)Cl, dans laquelle R représente un fluor ou un groupe alkyle perfluoré ayant 1 à 10 atomes de carbone, et X représente un chlore ou un fluor, procédé selon lequel on fait réagir en continu avec de l'oxygène, en phase gazeuse, avec addition de chlore élémentaire, des composés de formule RCFXCHCl₂, dans laquelle R et X ont le sens précité, en n'effectuant une irradiation d'activation qu'avec de la lumière ayant une longueur d'onde λ ≥ 290 nm.

2. Procédé selon la revendication 1, caractérisé en ce qu'on effectue la réaction dans un appareillage a circulation.

3. Procédé selon la revendication 2, caractérisé en ce qu'on introduit en continu la matière de départ et l'on retire en continu le produit de la réaction.

4. Procédé selon l'une des revendications précédentes, caractérisé en ce qu'on travaille sans pression.

5. Procédé selon l'une des revendications précédentes, caractérisé en ce que le rapport molaire entre le composé de départ RCFXCHCl₂ et le chlore élémentaire se situe dans le domaine de 100:1 à 3:1.

6. Procédé selon l'une des revendications précédentes, caractérisé en ce que le rapport molaire entre le composé de départ RCFXCHCl₂ et l'oxygène (O₂) se situe dans le domaine de 1:0,5 à 1:20.

7. Procédé selon l'une des revendications précédentes, caractérisé en ce que, pour préparer le chlorure de trifluoroacétyle, on part de 1,1-trifluoro-2,2-dichloroéthane (HCFC123), pour préparer le chlorure de chlorodifluoroacétyle on part du 1,1-difluoro-1,2,2-dichloroéthane (HCFC122) ou, pour préparer le chlorure de pentafluoropropionyle, on part du 1,1,1,2,2-pentafluoro-3,3- dichloropropane (HCFC225ca).

8. Procédé selon l'une des revendications précédentes, caractérisé en ce qu'on conduit la réaction à une température allant jusqu'à 200°C, avantageusement dans un intervalle de la température de 50 à 130°C.

9. Procédé selon l'une des revendications 2 à 8, caractérisé en ce que le temps de séjour moyen dans l'appareillage de circulation se situe entre 0,1 et 30 minutes.

10. Procédé selon l'une des revendications précédentes, caractérisé en ce qu'on utilise un émetteur de rayonnement à haute pression de Hg dopé par un iodure de métal.

11. Procédé selon la revendication 10, caractérisé en ce qu'on utilise un émetteur de rayonnement à haute pression de Hg dopé par de l'iodure de gallium, de l'iodure de thallium ou de l'iodure de cadmium.
